# EUROPEAN PATENT SPECIFICATION

(11) **EP 1 996 968 B1**
(45) Date of publication and mention of the grant of the patent: **08.03.2017**
(21) Application number: 07727175.7
(22) Date of filing: 21.03.2007
(51) Int. Cl.: G02B 3/14, A61F 2/16, A61F 9/00, G02C 7/04, G02C 7/06, G02B 26/00

(54) **INTRAOCULAR IMPLANT**
INTRAOKULARES IMPLANTAT
IMPLANT INTRAOCULAIRE

(30) Priority: 21.03.2006 EP 06300258
(43) Date of publication of application: 03.12.2008
(73) Proprietor: Parrot Drones, 75010 Paris (FR)
(72) Inventor: BERGE, Bruno, 69002 Lyon (FR); CRAEN, Pierre, 4053 Embourg (BE)
(74) Representative: Osha Liang
(86) International application number: PCT/EP2007/052699
(87) International publication number: WO 2007/107589

(56) References cited:
- WO-A-2004/077125
- WO-A-2005/088388
- US-A- 4 601 545
- US-A1- 2001 017 985
- US-A1- 2005 002 113
- US-B1- 6 369 954

## Description

### FIELD OF THE INVENTION

The present invention relates to an intraocular implant and in particular an intraocular implant having variable focus.

### BACKGROUND OF THE INVENTION

Ophthalmology intraocular implants are primarily used for people suffering from cataract (opacification of the crystalline). A current surgical technique involves removing the crystalline lens which has become opaque, and then reinserting an intraocular implant.

The intraocular implant is required because the removal of the crystalline changes the refractive power of the patient's eye, so that the eye can no longer focus. The intraocular implant aims at allowing the eye to focus at one particular distance, which is often chosen to be infinity. Usually these implants have lateral parts which serve to center the implant. In particular, the diameter of the cavity remaining after the crystalline removal is generally larger than the optical pupil diameter, and the lateral parts of the implant serve to maintain the implant at the centre of this cavity. This provides some alignment between the optical axis of the implant and the optical axis of the eye.

During an implant operation, the implant is generally inserted inside the patient's eye by making a small incision, typically a few millimetres across. The implant is made from polymers which are flexible enough to be folded or rolled up, such that prior to insertion, the implant is folded inside a holder. It is then inserted, via the small incision, into the eye's cavity, which has been left empty after removal of the crystalline lens. Once in position, the implant spontaneously comes back to its original unfolded shape, helped by its own elasticity.

A drawback of this surgery is that the patient loses any possibility of focusing at different object distances. The patient is then obliged to wear external lenses, for example contact lenses or glasses, for short distances, for example when reading, and to remove them for long distances.

Some implants have been developed to overcome the above mentioned difficulties, but they have a number of drawbacks. For example, multi-focal intraocular implants have been developed, which focus light at several fixed distances. However, a disadvantage is that the brain must then be trained to "use" this multi-focal lens, and this is time consuming for the patient and occasionally does not work. Furthermore, such multi-focal lenses produce intrinsically degraded image quality, resulting in unclear vision by the user.

Another attempt to resolve the problem of the lack of focusing is to use deformable intraocular implants, for which the action of the ciliary muscles can induce a focal change of the system. This type of implant is for example described in US2002/0193876 A1. According to this publication, when the ciliary muscles contract, the intraocular lens moves into an anterior position, and moves back to a posterior position when the muscles relax. However, in practise this system does not generally allow effective focusing.

Patent Application WO 2004/004605 A1 describes a general method of restoring automatic accommodation of a patient's eye, using pressure sensors which assess the converging power of the two eyes, and send a signal to actuators situated on the implants themselves. However, this document does not describe exactly how the focus of the implant can be varied, which is the key problem to be overcome.

Patent Application WO 2005/088388 A1 describes a variable focus lens for an eye comprising a transparent rear wall, a transparent front wall, a cavity formed between the transparent front wall and the transparent rear wall, first and second immiscible fluids of differing refractive index contained within said cavity, and electrodes to which a voltage is able to be applied to change the curvature of a fluid meniscus between the two fluids.

It is well known from specialists that the solutions described above do not give a satisfactory answer to the patient's problems: they do not provide the combination of a large amplitude accommodation (object distance 0.3m to infinity) with a good image quality on the retina, which would insure a comfortable vision to the patient.

### SUMMARY OF THE INVENTION

The present invention aims to provide an implant that at least partially addresses one or more drawbacks of prior art solutions.

The invention is defined by the appended claims.

According to a first aspect of the present invention, there is provided an intraocular variable focus implant comprising a flexible structure forming a pocket containing first and second transparent immiscible liquids, the first liquid being an insulating liquid and the second liquid being a conductive liquid, said liquids defining a liquid interface; and first and second electrodes, the first electrode being in contact with the second liquid and the second electrode being insulated from second conductive liquid by an insulating layer, wherein said liquid interface is moveable by electrowetting by a change in a voltage applied to between said first and second electrodes, wherein the flexible structure comprises a first flexible transparent film comprising a hydrophilic surface in contact with said second liquid and a second flexible transparent film comprising a hydrophobic surface in contact with said first liquid, wherein the first and second films are sealed to a third flexible film comprising a printed circuit, said printed circuit bearing electronic components for the control of the liquid lens, and wherein the first and second electrodes are mounted on the third film.

According to one embodiment of the present invention, the third film comprises a hydrophilic surface in contact with the second liquid.

According to one embodiments of the present invention, the second electrode is exposed on the outer surface of said structure such that when inserted in the eye it makes contact with intraocular serum of the eye, said second film forming said insulating layer.

According to embodiments of the present invention, a portion of the transparent film forms the insulating layer covering the second electrode.

According to embodiments of the present invention, the structure forms a second pocket filled with a second conductive liquid, the second conductive liquid forming the second electrode.

According to embodiments of the present invention, the intraocular implant further comprises centering means for optically centering the liquid interface in the structure.

According to embodiments of the present invention, the centering means comprises a surface of said insulating layer, said interface contacting said surface of the insulating layer, and said surface of the insulating layer being inclined with respected to an optical axis.

According to embodiments of the present invention, the intraocular implant further comprises control circuitry arranged to receive a focusing signal transmitted from a device external to the implant, the focusing signal representative of the distance at which it is desired for the eye to focus, and to apply a voltage between the electrodes of the liquid lens as function of the focusing signal.

According to embodiments of the present invention, the control circuitry comprises electronic components positioned on the flexible circuit.

According to embodiments of the present invention, the control circuitry comprises a miniaturized electric power unit, for on-site power generation.

According to alternative embodiments of the present invention, the control circuitry comprises power receiving circuitry for receiving power from an external power source.

Thus embodiments of the invention provide a flexible liquid lens and an active intraocular implant with said flexible liquid lens. The flexible liquid lens contains two liquids inside a flexible pocket, with transparent parts for the passage of the light from the exterior of the eye towards the retina, the liquids defining a liquid-liquid interface deformed by an electrowetting actuation. The two liquids have different refractive indices, one of them is conductive and the other one is insulating. It further comprises at least two electrodes, a first electrode being in contact with the conductive liquid and a counter electrode. A voltage applied between the electrodes will deform the interface between the two immiscible liquids inside the flexible pocket. Because the indices of refraction of the two liquids are different, the focus of the eye at different distances will be modified depending on the amplitude of the voltage.

The implant according to embodiments of the present invention also comprises control circuitry, made for example of a miniaturized electronic circuit, to detect a focusing signal from the outside of the eye, interpret this signal to set the focus at a given object distance, and produce an AC or DC voltage to change the shape of the liquid-liquid interface inside the pocket, so that the overall eye is focused at the said distance.

According to embodiments of the present invention, the control circuitry comprises an autonomous electronic system, including the power generating unit, which allows the implant to be buried inside the crystalline cavity without any physical connexion between the inside and the outside of the eye.

According to embodiments of the present invention, the liquid lens comprises centering means for the liquid interface, in order to maintain precisely the optical axis of the liquid surface aligned compared to the body of the intraocular implant. These centering means are for example internal features to maintain liquid interface centered inside the capsule. They should not be confused with the mechanical centering means which are at the outside of the capsule at the peripheral part of the implant, and which serve as a mechanical mean for centering the implant inside the intraocular cavity.

According to embodiments of the present invention, the liquid lens is made of deformable materials such as polymer sheets, bendable films, thin materials.

### BRIEF DESCRIPTION OF THE DRAWINGS

The foregoing and other purposes, features, aspects and advantages of the invention will become apparent from the following detailed description of embodiments, given by way of illustration and not limitation with reference to the accompanying drawings, in which:
Figures 1 to 5 are cross-section diagrams illustrating active intraocular implants according to a number of embodiments of the present invention; and
Figure 6 illustrates a commercial implant having a central optical part and lateral extensions, the lateral extension being outside the optical path, and serving as a mechanical centering means;
Figure 7 is a schematic plan view of the intraocular implant of Figure 1 according to an embodiment of the present invention; and
Figure 8 schematically illustrates circuitry for driving the liquid-liquid interface of the intra-ocular implant according to one embodiment of the present invention.

### DETAILED DESCRIPTION OF THE INVENTION

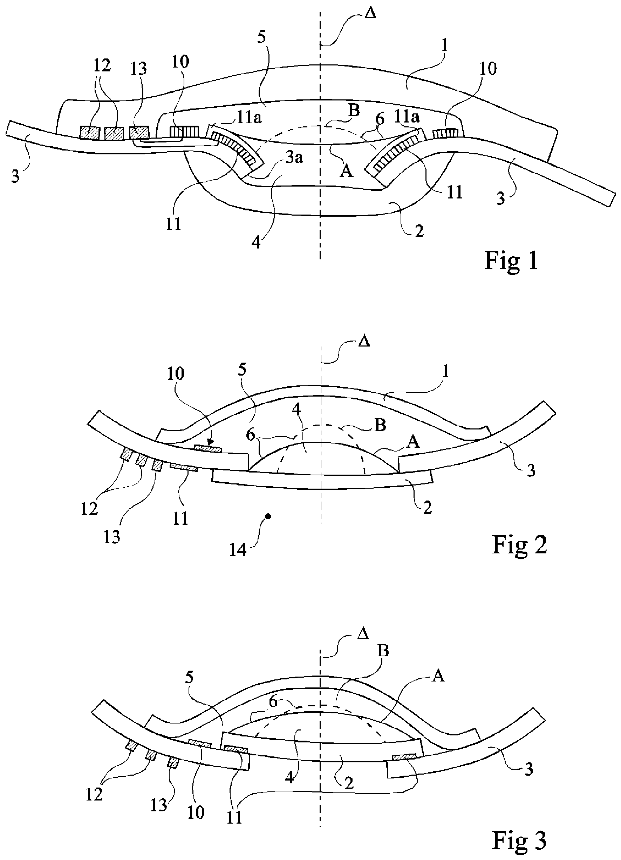

Figure 1 illustrates in cross-section an intraocular implant having a variable focus lens, the lens being variable by electrowetting, in particular by application of a voltage at a liquid-liquid interface as will be described in more detail below.

As illustrated, the implant comprises two flexible transparent films 1, 2 and a flexible circuit film 3, which in this example is in the shape of a flexible disk, having a hole passing through it such that it is annular. Together films 1, 2 and 3 form a flexible capsule or pocket. In particular, film 1 forms a flexible upper cap contacting the top surface of the film 3 in an annular band radially exterior to the hole through film 3. Film 2 forms a flexible lower cap contacting the underside of the film 3, in a band radially exterior to the hole through film 3.

These films 1, 2 and 3 serve as a container for two liquids, a non polar insulating liquid 4 forming a drop, which in this example comprises oil, and a conducting polar liquid 5, which in this example comprises water mixed with a salt. The liquids 4, 5 are immiscible with each other, and meet at an interface 6 which traverses the hole through the flexible film 3. The first and second liquids have different refractive indices, meaning that when the liquid-liquid interface 6 is curved, the interface forms a refractive surface, having an optical axis shown by dashed line Δ, which passes substantially through the middle of the hole of film 3. When the curvature of the refractive interface 6 changes, the refractive power also changes, allowing the eye in which the device is implanted to focus as different distances.

Electrodes 10 and 11 are provided mounted on the surface of the flexible circuit film 3. Film 3 is for example formed of a polyimide, such as materials known as Kapton or Mylar, the latter being a polyethylene terephthalate. In this embodiment, electrode 10 is annular in shape, and contacts liquid 5. Electrode 11 has a flat annular shape, and is covered by an insulating layer 11a, insulating it from contact with liquid 5. Electrodes 10 and 11 can for example be formed of Ni-gold. In this example electrode 11 covered by insulating layer 11a is formed on the top surface of film 3, in a band close to the inner edge 3a of film 3. The quantity of liquids 4, 5 are finely controlled and the inner surfaces of the films 1, 2 and 3 are formed such that the edge of the liquid-liquid interface 6 contacts with the surface of insulating layer 11a covering electrode 11.

By application of a voltage between electrodes 10 and 11, the contour of contact between the liquid-liquid interface 6 and the surface of insulating layer 11a can be varied, due to the electrowetting effect. The surface of insulating layer 11a is for example naturally or treated to be hydrophobic, discouraging contact with liquid 5, which as described above is a water based liquid in this example. Thus in an idle state in which no voltage is applied to the electrodes 10, 11, the interface 6 for example has a form shown by dashed line A in Figure 1, in which liquid 4 contacts most of the surface of the insulating layer 11a. However, when a voltage is applied between electrodes 10 and 11, the wettability of the surface of the insulating layer 11a by liquid 5 is increased, thereby causing liquid 5 to contact more of the surface of insulating layer 11a, and thereby moving the contour of contact between the liquid-liquid interface 6 and the insulating layer 11a radially inwards towards the optical axis Δ. At a maximum voltage, the interface for example has the form shown by dashed line B, in which liquid 5 contacts most of the surface of insulating layer 11a.

Electrowetting devices having a solid structure, but that comprise liquids movable according to the same principle as the implant of Figure 1, are for example described in European patent 1 166 157, in the name of the present Applicant.

The interface 6 can be centered at the optical axis Δ of the implant of Figure 1 by various techniques. In this embodiment it is centered by the inclined surface of the insulating layer 11a, which in this example is rotationally symmetric to the optical axis Δ. This shape for example results from the incline of the film 3 close to edge 3a, which is for example achieved by providing film 2 having a curved cross-section, and sealing the portion of film 3 close to edge 3a to the inner part of this curved portion. In this example, the surface of insulating layer 11a is not only inclined but also curved in cross-section, curving away from the optical axis Δ. At any given voltage applied between electrodes 10 and 11, there will be a preferred contact angle between the interface 6 and the surface of insulating layer 11a. If interface 6 moves off-center, the contact angle will increase at some parts of the edge of the interface 6, and decrease at others, thus causing a restoring force that re-centers the interface.

Alternatively or additionally, other techniques for centering the interface 6 could be used, including providing an insulating layer 11 having a graduated thickness moving out from the optical axis Δ, such that it is thinnest closest to the optical axis Δ close to edge 3a, and thicker radially outwards. Means for centering the liquid-liquid interface in a liquid lens are described in more detail in European Patent Application Nos. 98947621.3 and 00912738.2, which are hereby incorporated by reference to the extent allowable by the law.

The voltage applied to electrodes 10, 11 is for example an oscillating voltage (AC), but could also be a fixed voltage level (DC) in some embodiments. The voltage for example has a peak to peak value or amplitude controlled to be in the range of 0V to 40V, and/or an RMS value controlled to be in the range of 0V to 20V. However, in some embodiments, the highest applied voltage could be as low as 10V peak to peak to generate the maximum curvature of interface 6. While prior art solid liquid lenses have generally operated based on an oscillating supply voltage of around 60 V rms, it is possible to reduce this voltage to the values indicated above, for example by providing a slightly reduced focusing range, and a thinner insulating layer 11a. The frequency of the oscillating voltage is for example in the range 100 Hz to 10 kHz, and preferably approximately 1 kHz.

According to the present embodiment, the two transparent films 1 and 2 are hermetically sealed on the flexible circuit film 3, which is for example a flexible printed circuit. This circuit film for example comprises microelectronic circuits formed thereon. In particular the circuit film 3 comprises several electronic components 12 for receiving information from the outside of the eye, by any non-contact transmission, such as for example ultrasonic signals, infrared or radio communications, and according to some embodiments of the invention, also for receiving power. To generate the oscillating voltages applied between electrodes 10 and 11, components 12 on the top side of the flexible circuit film 3 receive the focusing signals from an external device, as explained in more detail below, and provide a signal to analog driving circuitry block 13 mounted on the top side of flexible circuit film 3, this block providing outputs to electrodes 10 and 11.

In operation, electronic components 12 receive information coming from an outside source that instructs the implant to focus at a particular distance, for example in order to focus on a relatively close object. The circuit 12 translates this information into a control signal, which is sent to the driver 13. Driver 13 generates the new drive voltage, which is for example an increased voltage if the new focusing distance is closer, or a lower voltage if the new focusing distance is further away, and this voltage is then applied between electrodes 10 and 11. The curvature of the liquid-liquid interface 6 changes directly in response to the changed voltage, for example from curvature A to B, or to any curvature in between these extremes, depending on the required focusing distance. The lens for example thus able to provide focusing at object distances from 0.3m to infinity.

In some embodiments electronic components 12, 13 could be made as a single component, depending on the particular microelectronic integration technique used.

When the implant of Figure 1 is to be inserted into the eye of a patient, this is performed in much the same way as described above in relation to prior art implants having fixed focus. In particular, the implant is generally inserted inside the patient's eye by making a small incision, typically a few millimetres across. Films 1, 2 and 3 are preferably formed of flexible inert materials known to be biocompatible, or materials coated with a biocompatible material. Films 1 and 2 could be transparent deformable films formed of materials such as PMMA (PolyMethylMetAcrylate) or FEP (FluoroEthyelenPropylene) films, having a thickness of between 1 and 100 µm. These films are flexible enough to be folded or rolled up, such that prior to insertion, the implant is for example folded inside a holder. As with the flexible fixed focus implants, it can then be inserted, via the small incision, into the eye's cavity, which has been left empty after removal of the crystalline lens. Once in position, the implant spontaneously comes back to its original unfolded shape, helped by its own elasticity.

To avoid the liquids 4 and 5 moving out of position when the lens is rolled up, or later in case of abrupt movements during use, the surfaces within the implant in contact with liquids 4 or 5 are preferably naturally or treated to be very hydrophobic or hydrophilic. For example, the inner surface of film 2 in contact with liquid 4 and the inner edge 3a of film 3 are preferably formed of a transparent material or have a transparent coating that is very hydrophobic. Possible surface coatings include PTFE (Polytetrafluoroethylene), commonly known by the brand name Teflon, or silicon products. The inner surface of film 1 in contact with liquid 5, and the surface of film 3 in contact with liquid 5 in Figure 1 are preferably formed of a transparent material or comprise a transparent surface coating that is naturally or treated to be very hydrophilic. Examples of materials that could be used are PMMA (Polymethylmethacrylate), or films formed of polyethylene, poly vinyl chloride or polyvinylidene fluoride, which have received a Corona treatment to give the surface a hydrophilic nature.

Films 1, 2 and 3, although formed of materials that provide a very good barrier to liquids, are preferably slightly permeable to fluid such as water. This means that in the long term, there will be an osmotic equilibrium between the inside and the outside of the implant. Advantageously liquids 4 and 5 are designed to be slightly more hygroscopic than the serum in which the implant will be immersed. This will tend to result in water from the eye serum penetrating into the implant up to a point at which a slight over-pressure is present in the implant, slightly inflating the implant. This helps to ensure that the implant does not dry out in the long term (which would cause fringes or defects), and maintains a consistent shape whereby all the polymer films 1, 2 and 3 are stretched. Assuming the polar liquid 5 is based on a water solution, this solution for example contains either hygroscopic solutes such as calcium chloride, cesium fluoride, potassium acetate, lithium chloride or bromide, zinc bromide, sodium bromide, magnesium chloride, ethylene glycol, mono propylene glycol, glycerol, 1,3 propane diol, or ethanol.

Another possibility is for the liquid 5 to contain the same salts as in the serum solution of the eye, which will be fluid directly outside the implant. Such salts are sodium chloride and potassium chloride, and are for example provided in the implant at slightly higher concentrations than they are present in the eye serum. This will again help to ensure a slight force tending to drive the water inside the implant by osmotic forces.

Figures 2 to 5 will now be described, which are all cross-sections illustrating alternative embodiments of variable liquid implants. The same or equivalent features in these figures have been referenced with the same numerals as those features in Figure 1.

The implant of Figure 2 is a variation of the design of that of Figure 1 that uses the fact that when in position in the eye the implant is immersed in the intraocular serum which is known to be a conductive solution (salted water). Thus in this embodiment, electrode 10 will contact the polar conducting liquid 5, as in the embodiment of Figure 1, while the other electrode 11 is a small electrode contacting with the serum 14 which is inside the eye, thereby forming the counter electrode. This means that electrical currents (although of a very small intensity) will circulate outside the implant.

In the embodiment of Figure 2, interface 6 contacts directly with the film 2. In this embodiment the liquid-liquid interface 6 between the liquids 4, 5 contacts the inner surface of film 2 directly, and is for example centered by using a graduated thickness for film 2, as described above. When no voltage is applied, the interface for example moves to the edge of the exposed surface of film 2, and moves no further due to surface wettabilities of the other surfaces in the pocket. In the embodiment of Figure 2, electrode 10 is mounted on the opposite face of the flexible circuit 3 to the other electronic components, but alternatively comments 12 and 13 could be mounted on the same side as electrode 10. Electrode 11 can cause electrowetting changes at interface 6, because the voltage will be carried by the naturally conductive serum present in the interior volume of the eye, in which the implant is immersed.

Figure 3 shows another embodiment wherein the transparent film 2 serving as a window is covering an annular electrode 11 on the inside of the flexible circuit film 3. This embodiment is similar to that of Figure 1, except that film 2 is sealed to the same side of film 3 as film 1, in these figures the top surface, such that film 2 itself provides the insulating layer between the electrode 11 and liquid 5. The design of Figure 3 could be slightly modified to have all electronic components on the same side of the flexible circuit, for example all on the top side.

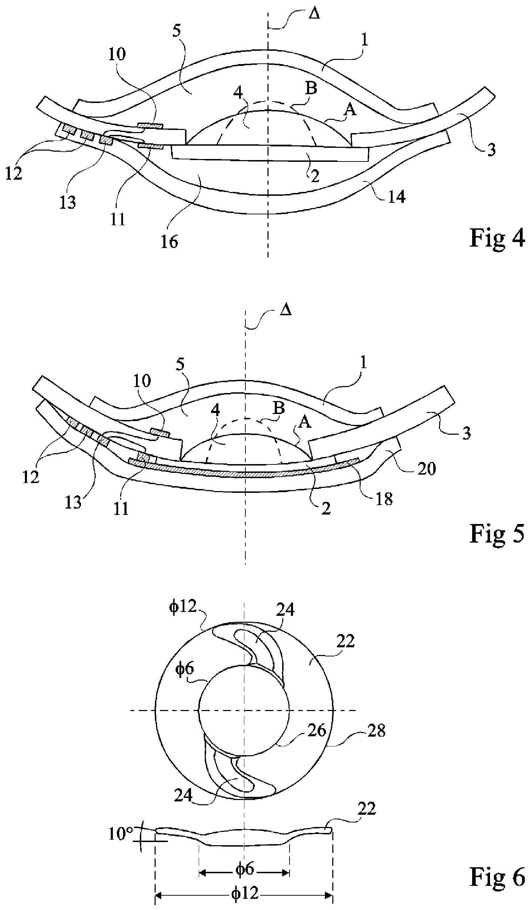

Figure 4 shows a variation of the embodiment of Figure 2 with an additional transparent film 14 creating a pocket of fluid 16 in contact with the outer surface of film 2, this fluid thus acting as the counter electrode, such that current no longer flows outside the implant in the serum of the eye. Liquid 16 is thus a polar conducting liquid in contact with solid electrode 11. The implant of Figure 4 thus has 2 liquid chambers.

Figure 5 shows another embodiment of the implant, again similar to the embodiment of Figure 2, except that a transparent electrode 18 has been formed covering the outer surface of film 2, providing the counter electrode. A further deformable film 20 has been formed covering the transparent electrode 18 and electronic components 12 and 13. Film 2 is for example now a very thin insulating layer that could be deposited over the transparent electrode 18, the transparent electrode being deposited on film 20. Thus thin electrode 18 and film 2 can be formed simply by a double coating process on film 20. Film 2 and electrode 18 are for example fixed in position by sealing film 20 to the flexible circuit film 3.

As an alternative, the electrode film 18 can be made from a non transparent material, if it for example deposited in the form of a ring, leaving the central region clear. By the central region, it is meant the smaller diameter of the liquid-liquid interface, corresponding on the Figure 5 to the base of the drop when it has the shape B.

With reference now to all the embodiments of Figure 1 to 5, it will be understood that the material and shape used for the external surfaces of the implant should generally be biocompatible with the inside of the eye and with the serum which are present. This implies that the implant should be of a compatible shape, for instance as described in more detail in WO 2004/069101 A1, which is hereby incorporated by reference to the extent allowable by the law. Furthermore, the material can for example be chosen to be PMMA, in order to avoid any potential problems such as for example implant reject or opacification.

In all realizations of implants described herein, features of the design are preferably chosen to give a precise optical power of the implant when no voltage is applied to the electrodes 10, 11, such that even when the implant receives no power, focusing by the eye is possible, for example at infinity. This can be achieved in part by giving some optical power to the films 1, 2. For example, the embodiment of Figure 1 illustrates film 1 having a lens shape. Furthermore, the external shape of the implant and radii of curvature can be controlled, or flexible films 1 and 2 could be replaced by semi-flexible lens shape windows (not shown on the figures), allowing different patients to have different power ocular correction. However, implants are preferably always adapted to a patient's eyes to give focus at infinity when no voltage is applied to the device.

According to some embodiments, the implants of Figure 1 to 5 further comprise mechanical centering means (not shown in the Figures) which serve to centre the implant in the cavity of the eye left after the removal of the crystalline lens. These mechanical centering means will be described with reference to Figure 6.

Figure 6 illustrates, in plan view and cross-section, a known commercial implant, and will be used to illustrate an example of an exterior shape of the lens that encourages it to be centered. In particular, the commercial implant 22 comprises protruding curved ridges 24, formed spiralling outwards from an inner diameter 26 of the lens towards the outer edge 28 of the lens. Two such ridges are provided in this example, at opposite sides of the lens. The embodiments of Figures 1 to 5 preferably comprise such ridges. Alternatively, there are numerous other known designs of similar centering means that could be incorporated in these implant embodiments to help centering the implant.

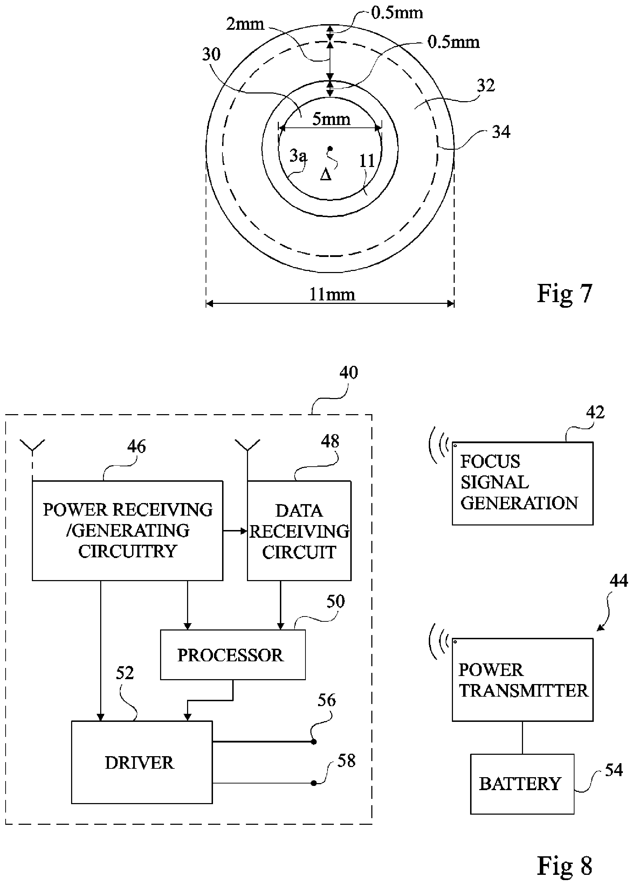

Figure 7 illustrates the implant of Figure 1 in plan view, however the illustration of Figure 7 is very similar to if not the same as a plan view taken of any of the embodiments in Figures 1 to 5. Although not shown in Figure 7, the implant preferably also comprises the ridges 24 shown in Figure 6. As illustrated, the implant is circular in plan view, with an opening 30, which is the opening through flexible circuit film 3, giving it its annular shape. In this example this opening is 5mm in diameter, this being the effective lens diameter in this embodiment. Electrode 11 is provided in an annular region on the annular film 3 extending from the edge of the opening 3a, which is at a radius of 2.5 mm from the center of the implant (optical axis Δ) to a radius approximately 3 mm from the centre of the implant. In this embodiment, the implant is 11 mm in diameter. Assuming that an outer region 0.5 mm in diameter is free from electronics, this leaves a region 32 between electrode 11 and the dashed line 34 in Figure 7 in which electronics for receiving focus information signals, generating lens driving signals and driving the lens can be incorporated. Such an annular band having a width of 2 mm and outer diameter of 10 mm has an area of over 50 mm², which is a relatively large area in which circuitry for driving the lens can be mounted.

Figure 8 illustrates schematically examples of the circuit blocks 40 within the implant and blocks 42, 44 external to the implant, for driving the liquid interface.

Within the implant, mounted on or within flexible circuit film 3, power receiving/generating circuitry 46, a data receiving circuit 48, processing means 50, and driving circuitry 52 are provided. External to the implant, a focus signal generation block 42 is provided, that transmits a focus signal to the data receiving circuit. Such a block is for example positioned and arranged to sense movement of muscles close to the eye that contract or relax in order to focus the eye. In this way, focusing can be provided by the brain directly, and interpreted and transmitted to the implant. Miniature RF transmitters and receivers are well known in the art, and provide one example of a possible wireless connection between block 42 and circuit 48. As an alternative, block 42 could be part of an external auto-focusing device, which is for example attached to glasses worn by the patient and pointed at objects that the patient wishes to focus on. Focusing information can then be transmitted in the same way. Alternatively, block 42 could be a manual wireless remote control operated by the patient, allowing the patient to choose focusing themselves.

In some embodiments, the implant is able use an internal self-contained power source within the eye. For example, power could be taken from muscles around the eye, or from movements of the eye. In this case, the power circuit is a generating circuit that generates a voltage from the power source. Alternatively, power is transmitted to the device from an external source, in a similar fashion to the power transmitted to an RF tag. Such an external source could be a battery 54, or alternative source, and could be inserted elsewhere in the patient's body.

The power circuitry is connected to all other circuit blocks in the device to provide them with power.

Processing means 50 is for example a micro-processor chip that receives the focusing data received from the data receiving circuit 48, and calculates the required drive voltage for obtaining this focal distance. Lookup tables or the like are for example used to determine the drive voltage.

The driving circuitry receives the required drive voltage information from processor 50, which is for example in the form of a low voltage signal, which is then amplified by the driving circuitry 52. The driving circuitry outputs a voltage between two output lines 56 and 58 which are connected to the electrodes 10 and 11 respectively.

Having thus described at least one illustrative embodiment of the invention, various alterations, modifications and improvements will readily occur to those skilled in the art.

For example, it will be apparent that features described in relation to one or more of the embodiments could be incorporated in any combination in other embodiments of the implant.

Such alterations, modifications and improvements are intended to be within the spirit and scope of the invention. Accordingly, the foregoing description is by way of example only and is not intended to be limiting. The invention is limited only as defined in the following claims and the equivalent thereto.

## Claims

1. An intraocular variable focus implant comprising:
a flexible structure forming a first pocket containing first and second transparent immiscible liquids, the first liquid being an insulating liquid (4) and the second liquid being a conductive liquid (5), said liquids defining a liquid interface (6),
said flexible structure comprising a first flexible transparent film (1) comprising a hydrophilic surface in contact with said second liquid, and a second flexible transparent film (2) comprising a hydrophobic surface in contact with said first liquid, and
said first and second films being sealed to a third flexible film so to form the first pocket, the third flexible film (3) comprising a printed circuit, said printed circuit bearing electronic components (12) for the control of the liquid lens; and
first and second electrodes (10, 11), the first electrode (10) being in contact with the second liquid and the second electrode (11) being insulated from second conductive liquid by an insulating layer (11a), wherein the first and second electrodes (10, 11) are mounted on the third film (3), and wherein said liquid interface is moveable by electrowetting by a change in a voltage applied to between said first and second electrodes.

2. The intraocular implant of claim 1, wherein the third flexible film comprises a hydrophilic surface in contact with the second liquid.

3. The intraocular implant according claim 1 or claim 2, wherein the second electrode (11) is exposed on the outer surface of said structure such that when inserted in the eye it makes contact with intraocular serum of the eye, said second film forming said insulating layer.

4. The intraocular implant according to claim 1 or claim 2, wherein a portion of the transparent film (2) forms the insulating layer covering the second electrode (11).

5. The intraocular implant according to claim 1 or claim 2, further comprising an addition layer (14) forming together with the second flexible transparent film (2) and the third flexible film (3) a second pocket filled with a third conductive liquid (16), wherein the second electrodes (11) are mounted on the side of the third flexible film (3) contacting the third conductive liquid (16), wherein the insulating layer insulating the second electrode (11) from the second conductive liquid (5) is provided by the third flexible film (3).

6. The intraocular implant according to any preceding claim, further comprising centering means for optically centering the liquid interface in the structure.

7. The intraocular implant of claim 6, wherein said centering means comprises a surface of said insulating layer, said interface (6) contacting said surface of the insulating layer (11a), and said surface of the insulating layer being inclined with respected to an optical axis (Δ).

8. The intraocular implant according to any preceding claim, further comprising control circuitry arranged to receive a focusing signal transmitted from a device (42) external to the implant, the focusing signal representative of the distance at which it is desired for the eye to focus, and to apply a voltage between the electrodes of the liquid lens as function of the focusing signal.

9. The intraocular implant according to claim 8, wherein the control circuitry comprises a miniaturized electric power unit, for on-site power generation.

10. The intraocular implant of claim 8, wherein the control circuitry comprises power receiving circuitry for receiving power from an external power source.

## Patentansprüche

1. Intraokulares Implantat mit variablem Fokus, das umfasst:
eine flexible Struktur, die eine erste Tasche bildet, die erste und zweite transparente unvermischbare Flüssigkeiten enthält, wobei die erste Flüssigkeit eine isolierende Flüssigkeit (4) ist und die zweite Flüssigkeit eine leitende Flüssigkeit (5) ist, wobei die Flüssigkeiten eine Flüssigkeitsgrenzfläche (6) definieren,
wobei die flexible Struktur einen ersten flexiblen transparenten Film (1), der eine hydrophile Oberfläche in Kontakt mit der zweiten Flüssigkeit umfasst, und einen zweiten flexiblen transparenten Film (2) umfasst, der eine hydrophobe Oberfläche in Kontakt mit der ersten Flüssigkeit umfasst, und
wobei der erste und der zweite Film mit einem dritten flexiblen Film versiegelt sind, um die erste Tasche zu bilden, wobei der dritte flexible Film (3) eine gedruckte Schaltung umfasst, wobei die gedruckte Schaltung elektronische Komponenten (12) zur Steuerung der Flüssiglinse aufweist; und
erste und zweite Elektroden (10, 11), wobei die erste Elektrode (10) mit der zweiten Flüssigkeit in Kontakt steht und die zweite Elektrode (11) durch eine Isolierschicht (11a) gegenüber der zweiten leitenden Flüssigkeit isoliert ist, wobei die ersten und zweiten Elektroden (10, 11) auf dem dritten Film (3) angebracht sind, und wobei die Flüssigkeitsgrenzfläche durch Elektrobenetzung durch eine Änderung einer Spannung bewegbar ist, die zwischen der ersten und der zweiten Elektrode angelegt wird.

2. Intraokulares Implantat nach Anspruch 1, wobei der dritte flexible Film eine hydrophile Oberfläche in Kontakt mit der zweiten Flüssigkeit umfasst.

3. Intraokulares Implantat nach Anspruch 1 oder 2, wobei die zweite Elektrode (11) auf der Außenfläche der Struktur freigelegt ist, sodass sie, wenn sie im Auge eingesetzt ist, mit dem intraokularen Serum des Auges in Kontakt gelangt, wobei der zweite Film die Isolierschicht bildet.

4. Intraokulares Implantat nach Anspruch 1 oder 2, wobei ein Abschnitt des transparenten Films (2) die Isolierschicht bildet, die die zweite Elektrode (11) abdeckt.

5. Intraokulares Implantat nach Anspruch 1 oder 2, das ferner eine Additionsschicht (14) umfasst, die gemeinsam mit dem zweiten flexiblen transparenten Film (2) und dem dritten flexiblen Film (3) eine zweite Tasche bildet, die mit einer dritten leitenden Flüssigkeit (16) gefüllt ist, wobei die zweiten Elektroden (11) auf der Seite des dritten flexiblen Films (3) in Kontakt mit der dritten leitenden Flüssigkeit (16) angebracht sind, wobei die Isolierschicht, die die zweite Elektrode (11) gegenüber der zweiten leitenden Flüssigkeit (5) isoliert, durch den dritten flexiblen Film (3) bereitgestellt wird.

6. Intraokulares Implantat nach einem vorstehenden Anspruch, das ferner ein Zentriermittel zum optischen Zentrieren der Flüssigkeitsgrenzfläche in der Struktur umfasst.

7. Intraokulares Implantat nach Anspruch 6, wobei das Zentriermittel eine Oberfläche der Isolierschicht umfasst, wobei die Crrenzfläche (6) die Oberfläche der Isolierschicht (11a) kontaktiert und die Oberfläche der Isolierschicht in Bezug auf eine optische Achse (Δ) geneigt ist.

8. Intraokulares Implantat nach einem vorstehenden Anspruch, das ferner einen Steuerschaltkreis umfasst, der so ausgelegt ist, dass er ein Fokussiersignal empfängt, das von einer in Bezug auf das Implantat externen Vorrichtung (42) gesendet wird, wobei das Fokussiersignal für den Abstand repräsentativ ist, in dem der Fokus des Auges erwünscht ist, und dass er eine Spannung zwischen den Elektroden der Flüssiglinse als Funktion des Fokussiersignals anlegt.

9. Intraokulares Implantat nach Anspruch 8, wobei der Steuerschaltkreis eine miniaturisierte elektrische Leistungseinheit für eine Vor-Ort-Leistungserzeugung umfasst.

10. Intraokulares Implantat nach Anspruch 8, wobei der Steuerschaltkreis einen Leistungsaufnahmeschaltkreis zum Aufnehmen von Leistung von einer externen Leistungsquelle umfasst.

## Revendications

1. Implant à focalisation variable intraoculaire, comprenant :
une structure flexible formant une première poche contenant des premier et deuxième liquides transparents non miscibles, le premier liquide étant un liquide isolant (4) et le deuxième liquide étant un liquide conducteur (5), lesdits liquides définissant une interface liquide (6),
ladite structure flexible comprenant un premier film flexible transparent (1) comprenant une surface hydrophile en contact avec ledit deuxième liquide, et un deuxième film flexible transparent (2) comprenant une surface hydrophobe en contact avec ledit premier liquide, et
lesdits premier et deuxième films étant scellés à un troisième film flexible de manière à former la première poche, le troisième film flexible (3) comprenant un circuit imprimé, ledit circuit imprimé supportant des composants électroniques (12) pour la commande de la lentille liquide ; et
des première et deuxième électrodes (10,11), la première électrode (10) étant en contact avec le deuxième liquide et la deuxième électrode (11) étant isolée du deuxième liquide conducteur par une couche isolante (11a), dans lequel les première et deuxième électrodes (10,11) sont montées sur le troisième film (3), et dans lequel ladite interface liquide est déplaçable par électromouillage par un changement dans une tension appliquée entre lesdites première et deuxième électrode.

2. Implant intraoculaire selon la revendication 1, dans lequel le troisième film flexible comprend une surface hydrophile en contact avec le deuxième liquide.

3. Implant intraoculaire selon la revendication 1 ou la revendication 2, dans lequel la deuxième électrode (11) est exposée sur la surface extérieure de ladite structure de sorte que, lorsqu'elle est insérée dans l'oeil, elle entre en contact avec un sérum intraoculaire de l'oeil, ledit deuxième film formant ladite couche isolante.

4. Implant intraoculaire selon la revendication 1 ou la revendication 2, dans lequel une partie du film transparent (2) forme la couche isolante recouvrant la deuxième électrode (11).

5. Implant intraoculaire selon la revendication 1 ou la revendication 2, comprenant en outre une couche additionnelle (14) formant ensemble avec le deuxième film flexible transparent (2) et le troisième film flexible (3) une deuxième poche remplie d'un troisième liquide conducteur (16), dans lequel les deuxièmes électrodes (11) sont montées sur le côté du troisième film flexible (3) contactant le troisième liquide conducteur (16), dans lequel la couche isolante isolant la deuxième électrode (11) du deuxième liquide conducteur (5) est fournie par le troisième film flexible (3).

6. Implant intraoculaire selon une quelconque des revendications précédentes, comprenant en outre un moyen de centrage pour centrer optiquement l'interface liquide dans la structure.

7. Implant intraoculaire selon la revendication 6, dans lequel ledit moyen de centrage comprend une surface de ladite couche isolante, ladite interface (6) contactant ladite surface de la couche isolante (11a), et ladite surface de la couche isolante étant incliné par rapport à un axe optique (Δ).

8. Implant intraoculaire selon une quelconque des revendications précédentes, comprenant en outre un circuit de commande agencé pour recevoir un signal de focalisation transmis à partir d'un dispositif (42) externe à l'implant, le signal de focalisation étant représentatif de la distance à laquelle il est souhaité pour l'oeil de focaliser, et pour appliquer une tension entre les électrodes de la lentille liquide en fonction du signal de focalisation.

9. Implant intraoculaire selon la revendication 8, dans lequel le circuit de commande comprend une unité d'alimentation électrique miniaturisée, pour une génération d'alimentation électrique sur place.

10. Implant intraoculaire selon la revendication 8, dans lequel le circuit de commande comprend un circuit de réception d'alimentation électrique pour recevoir une alimentation électrique provenant d'une source d'alimentation électrique externe.
